Europäisches Patentamt

European Patent Office

Office européen des brevets

(19)

(11) Veröffentlichungsnummer: **0 140 134**
**B1**

(12) **EUROPÄISCHE PATENTSCHRIFT**

(45) Veröffentlichungstag der Patentschrift: **06.06.90**

(51) Int. Cl.⁵: **A 61 K 35/12**

(21) Anmeldenummer: **84111170.1**

(22) Anmeldetag: **19.09.84**

(54) **Verfahren zur Herstellung eines biologisch aktiven Extraktes.**

(30) Priorität: **05.10.83 CH 5413/83**

(43) Veröffentlichungstag der Anmeldung:
**08.05.85 Patentblatt 85/19**

(45) Bekanntmachung des Hinweises auf die
Patenterteilung:
**06.06.90 Patentblatt 90/23**

(84) Benannte Vertragsstaaten:
**AT BE CH DE FR GB IT LI NL SE**

(56) Entgegenhaltungen:
**DE-A-2 325 196**
**FR-A-2 439 205**
**FR-A-2 487 197**

**CHEMICAL ABSTRACTS, Band 96, Nr. 7, 15.
Februar 1982, Seite 501, Spalte 2,
Zusammenfassungsnr. 50982z, Columbus, Ohio,
US;**

(73) Patentinhaber: **Solco Basel AG
Gellertstrasse 18
CH-4052 Basel (CH)**

(72) Erfinder: **Oertli, Robert
Bremenstallstrasse 21
CH-4313 Möhlin (CH)**

(74) Vertreter: **Frossard, Michel, Dr. et al
A. Braun, Braun, Héritier, Eschmann AG,
Patentanwälte Holbeinstrasse 36-38
CH-4051 Basel (CH)**

## Beschreibung

Verfahren zur Herstellung von Organextrakten sind seit längerer Zeit bekannt. Verschiedene Autoren begnügten sich mit einer einfachen Extraktion meistens einer wässrigen Lösung, oft blieb es sogar bei einer mechanischen Bearbeitung des Organs, dies vor allem in rein wissenschaftlichen Arbeiten. Die zunehmende Bedeutung der Transplantation von Knochenmark, Niere, Herz, Blut usw. und die Erkenntnis der biologischen Aktivität der Organe, speziell des Thymus (Hess, 1968) im Zusammenhang mit der raschen Entwicklung der zellulären Immunologie bewirkten eine intensive Forschung nach Methoden und Verfahren zur Herstellung von biologisch wirksamen Organextrakten zur therapeutischen Anwendung. Je nach den Anforderungen an das Präparat werden bei der Herstellung biologisch schlecht verträgliche Mittel wie Säuren, Aceton oder auch Alkohol eingesetzt. Die Produkte unterscheiden sich im wesentlichen von einem Gesamtextrakt dadurch, dass es sich entweder um eine reine, genau charakterisierte Verbindung oder um ein analytisch genau definiertes Gemisch von Substanzen des entsprechenden Organs handelt.

Gegenstand der vorliegenden Erfindung hingegen ist ein Verfahren zur Herstellung eines biologisch aktiven, salzarmen, pyrogenfreien und antigenfreien Gesamtextraktes aus Organen von Säugetieren oder Zellkulturen. Der Extrakt besteht aus einem Gemisch biologisch wirksamer Substanzen mit einem Molekulargewicht unterhalb 10'000 Daltons und zeichnet sich insbesondere dadurch aus, dass er, ausser Wasser, überhaupt keine fremden Stoffe oder Hilfsstoffe oder sonstwie zugesetzten Substanzen enthält.

Es wurde nämlich überraschenderweise gefunden, dass die zur Herstellung von Organextrakten bisher üblichen Fällungsreaktionen gänzlich ausgelassen und mit Vorteil durch Hitzendenaturierung in einem Wärmeaustauscher mit hintereinander arbeitenden Wärmeträger und Kühlmedium ersetzt werden können.

Das neue Verfahren besteht also darin, dass man

a) das unter möglichst keimarmen, für Zellkulturen sterilen Bedingungen beschaffene Ausgangsmaterial unter Aufschluss der Zellen zerkleinert,

b) das zerkleinerte und aufgeschlossene Material in einem als Wäremeaustauscher fungierenden Durchlaufsystem mit hintereinander arbeitendem Wärmeträger und Kühlmedium rasch auf eine Temperatur von 70 bis 90°C,

vorzugsweise auf etwa 80°C, erhitzt und nach beliebiger Zeitdauer rasch wieder auf niedrige Temperatur, z.B. 4 oder 10°C, abkühlt, wodurch einerseits thermolabile Bestandteile, wie z.B. Proteine, ausgefällt und andererseits die Bakterien und Enzyme inaktiviert werden, ohne dass sie in dem mittleren Temperaturbereich wieder proliferieren bzw. aktiv werden konnten,

c) die ausgefällten Produkte durch Zentrifugieren von der Lösung abtrennt,

d) in der Lösung verbliebene Substanzen mit einem Molekulargewicht über 10'000 Daltons durch Ultrafiltration entfernt, wodurch die Antigene und Endotoxine eliminiert werden, und

e) der verbleibenden Lösung durch Elektrodialyse die gelösten Salzionen weitgehend entzieht.

Obschon der erhaltene Extrakt von dem Herstellungsverfahren her bereits steril und pyrogenfrei ist, kann man gewünschtenfalls als Sicherheitsmassnahme eine Sterilfiltration anschliessen.

In scharfem Gegensatz zu den bisher bekannten Verfahren wird gemäss vorliegender Erfindung überhaupt kein fremder Stoff — ausgenommen Wasser — in das System eingeführt. Insbesondere werden dem Ausgangsmaterial, den verschiedenen Zwischenprodukten und dem Endprodukt weder Konservierungsmittel, noch irgendwelche chemische Zusätze, wie z.B. Bakterizide (z.B. Azide), welche üblicherweise die Sterilität und Pyrogenfreiheit gewährleisten, zugegebenen: der erhaltene Extrakt ist aufgrund der Herstellungsart pyrogenfrei und steril. Das Ausbleiben jeglicher Fremdstoffzugabe im Verlauf des Verfahrens bringt für den Patienten den Vorteil einer erhöhten Sicherheit, für die Registrierungsbehörde den Vorteil einer leichteren Beurteilung, für den Hersteller den Vorteil eines geringeren Aufwands mit sich.

Besonders erwähnenswert beim erfindungsgemässen Verfahren ist der bewusste Verzicht auf die Verwendung jedwelchen Trägermaterials bzw. auf die Anwendung jeglicher säulenchromatographischen Trennung. Auch dadurch steht das neue in klarem Gegensatz zu den bekannten Extraktionsverfahren; das Verfahrensprodukt enspricht denn auch in seiner Zusammensetzung diesem bedeutsamen Unterschied.

Werden nämlich die Substanzen des Extraktes nach ihrem grundlegenden physikalisch-chemischen Verhalten in Gruppen eingeteilt, so ergibt sich für den verfahrensgemässen Extrakt folgendes Bild:

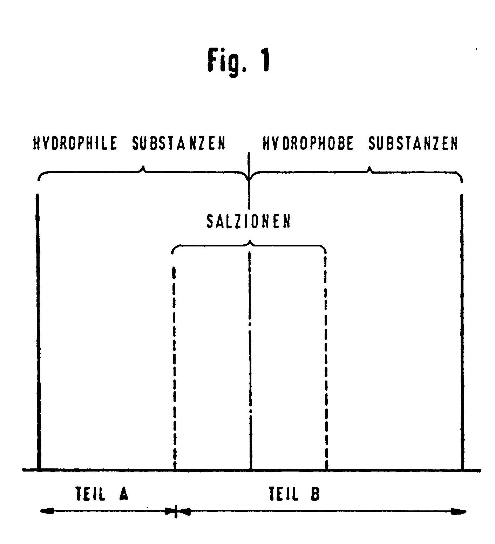

1. Gruppe:  hydrophile Substanzen (mehrheitlich Ninhydrin-positiv)  Teil A in Fig. 1

2. Gruppe:  Salzionen;

3. Gruppe:  hydrophobe Substanzen (mehrheitlich Anisaldehyd-positiv)  } Teil B in Fig. 1

Schematisch dargestellt sieht die Verteilung der drei Gruppen wie in der beiliegenden Fig. 1 aus.

Bei einer säulenchromatographischen Enfernung der zum Teil unerwünschten Salzionen, z.B. über Sephadex (R), verbleibt im Endprodukt lediglich der mit A bezeichnete Teil der Substanzen, während der Teil B, d.h. nebst den Salzionen sämtliche hydrophoben Substanzen und sogar auch zum Teil Ninhydrinpositive Substanzen, verloren geht. Nun besitzen Substanzen aus dem Teil B nachweislich eine biologische Wirkung; ihr Verlust bedeutet daher für das entsprechende Präparat einen empfindlichen Nachteil gegenüber dem Gesamtextrakt aus dem vorliegenden Verfahren.

Ausserdem wird durch eine Säulenchromatographie ein fremder Stoff, nämlich das Trägermaterial, in das System eingeführt. Dadurch erfolgt in der Regel die Gefahr einer bakteriellen Kontamination des Produktes, welche zu pyrogenhaltigen Endprodukten führt, während das erfindungsgemäs erhaltene Endprodukt pyrogenfrei ist.

Schliesslich entfallen, durch den Verzicht auf jegliche chromatographische Trennung, einerseits der mit der Elution einhergehende grosse Zeitaufwand und andererseits der Arbeitsaufwand, welcher anschliessend zur Entfernung der verwendeten grossen Mengen Elutionsmittel (Wasser) notwendig ist.

Erhalten wird also erstmals ein Gesamtextrakt, welcher — verglichen mit den Produkten der bereits bekannten Extraktionsverfahren — alle biologisch aktiven Substanzen, insbesondere alle niedermolekularen (< 10'000 Daltons) Peptide, enthält und zugleich infolge seines geringen Salzegehaltes und seiner Pyrogen- und Antigenfreiheit direkt, ohne weitere Massnahmen verabreicht werden kann.

Bedenkt man die Schwierigkeiten und den Arbeitsaufwand, welche die Entfernung pyrogenen Materials aus parenteral zu verabreichenden biologischen Produkten in der Regel verursacht (siehe z.B. deutsche Offenlegungsschrift 3 229 132), bedenkt man ferner die vielen sensiblisierenden oder schädigenden Einflüsse der üblicherweise zugesetzten Konservierungsmittel (siehe u.a. Hagers Handbuch der pharmazeutischen Praxis, Springer Verlag, Berlin Heidelberg New York 1977, Seite 313), dann werden die Besonderheit des neuen Verfahrens und seine Ueberlegenheit gegenüber den bekannten Extraktionsverfahren erst recht offensichtlich.

Im folgenden wird das Verfahren ausführlich beschrieben.

Das Verfahren lässt sich zur Herstellung von allen möglichen organischen Extrakten anwenden. Die wichtigsten Ausgangsprodukte sind folgende Organe und Gewebe: Milz, Thymus, Leber, Herz, Plazenta, Knochenmark und Blut. Es können aber acuh Extrakte aus Zellkulturen, insbesondere aus Kulturen von tierischen Zellen, hergestellt werden, beispielsweise aus Kulturen von Thymozyten (Thymuszellen), von fetalen Kälbernierenzellen, von Leukozyten, von Hybridzellen, von Fibroblasten usw. Selbstverständlich können nicht nur die Zellkulturen als solche, sondern auch gewaschene Zellmassen aus den entsprechenden Kulturen als Ausgangsmaterial eingesetzt werden.

Das Verfahren beschränkt die Herkunft und die Vorbehandlung des Ausgangsmaterials nicht. Die biologische Wirkung des Extraktes ist nicht auf eine Substanzklasse oder eine Indikation beschränkt. Der aus diesem Verfahren entstandene Extrakt kann durch weitere Stufen in Fraktionen aufgetrennt und es können daraus reine Substanzen isoliert werden.

a) Zerkleinerung und Aufschluss der Zellen

Die Beschaffung der Organe bzw. der Zellkulturen erfolgt jeweils unter Einhaltung strenger vorschriften für keimarme bzw. sterile Arbeitsweise. Im Falle von tierischen Organen wird das entfettete Material unmittelbar nach seiner Gewinnung aufgearbeitet, d.h. zerkleinert und aufgeschlossen. Wenn dies nicht sofort möglich ist, wird es tiefgekühlt, z.B. auf −25°C oder auch in flüssigem Stickstoff, und in tiefgekühltem Zustand bis zu seiner Aufarbeitung gelagert; dadurch werden die enthaltenen Bakterien und Enzyme inaktiv. Die allenfalls tiefgefrorenen Organe werden dann in einem Fleischwolf zu einem feinen Brei zerhackt und die Zellen durch Verdünnen 1:1 (Gewicht/Volumen) mit pyrogenfreiem, sterilem und bidestiliertem Wasser aufgeschlossen.

Im Falle von Zellkulturen sollen die Beschaffung und allenfalls die Lagerung unter sterilen Bedingungen erfolgen. Die Zerkleinerung und der Aufschluss werden vorzugsweise durch Einwirkung von Ultraschall ausgeführt. Die Ultraschallbehandlung soll unter Kühlung, vorzugsweise bei einer Temperatur von etwa 0 bis etwa 10°C, erfolgen.

b) Proteinfällung durch Hitze

Der Brei wird in einem Durchlaufsystem erhitzt und anschliessend in einem zweiten gleichen System abgekühlt. Der Brei fliesst dabei durch ein spiralenförmig angeordnetes Stahlrohr, welches in einem vom Wärmeträger bzw. Kältemedium durchströmten Mantelrohr steckt. Die Durchflussgeschwindigkeit des Breis kann kontrolliert werden. Gegenüber dem herkömmlichen Erhitzen im Rundkolben kann wesentlich Zeit eingespart und eine reproduzierbare Proteinfällung erreicht werden.

Diese Art und Ausführungsweise der Hitzedenaturierung bringt in der Tat verschiedene spezifische Vorteile mit sich. Durch den effektiven und schnellen Wärmeaustausch des Wärme- bzw. Kälte-Mediums auf das Produkt, welches seinerseits durch ein Mischsystem im Rohr ständig gemischt wird, so dass alle Teilchen gleichmässig erwärmt werden, ist es möglich beliebig grosse Mengen kontinuierlich zu bearbeiten.

Das Baukastensystem des Wärmeaustauschers ermöglicht jeweils mehrere Bedingungen der Empfindlichkeit des Präparates bzw. den Anforderungen anzupassen.

regulierbare Temperatur,

regulierbare Verzweilzeit bzw. Reaktionszeiten,

die Temperatursteigerung kann auch stufenweise erfolgen.

Diese Bauweise erlaubt also eine schonende und sowohl physikalisch als auch biochemisch optimale Behandlung des Produktes.

Der rasche Temperaturaustausch verkürzt die Arbeitszeit, z.B. die Zeit für die Hitzedenaturierung, um 75% und mehr gegenüber der herkömmlichen Methode mit dem Rundkolben. Dadurch wird nicht nur Zeit, sondern durch den besseren Wirkungsgrad auch Energie gespart.

c) Abtrennung des ausgefällten Materials

Die Trennung der festen und flüssigen Phase erfolgt durch Zentrifugation, mit Vorteil in einem Dekanter. Da mit einer Laborzentrifuge nur relativ kleine Mengen (für 10 Liter werden 2 volle Stunden benötigt) getrennt werden können, hat sich die Anwendung eines Durchflusssystems sowohl für diesen Arbeitsvorgang als auch für eine kontinuierliche Produktion besonders vorteilhaft erwiesen. Zum Beispiel benützt man den Schnelldekanter von Flottweg Werk (Dr. Georg Bruckmayer GmbH & Co. KG, Vilsbiburg, BRD). Während die Feststoffe für Nebenprodukte Verwendung finden können, wird die flüssige Phase der nächsten Stufe zugeführt.

d) Ultrafiltration mit einem Hohlfaser-System

Die geklärte wässrige Lösung aus dem Dekanter wird sukzessiv beispielsweise mit 3 Volumen pyrogenfreiem, sterilem und bidestilliertem Wasser versetzt und ultrafiltriert. Die Ultrafiltration kann auch bei kontinuierlicher Zugabe des mehrfachen Volumens Wasser erfolgen. Dies hat den Vorteil, dass unter möglichst gleichen Bedingungen eine optimale Ausbeute an Anteilen von MG <10'000 durch die Filtration erreicht wird. Auch durch Regulierung der Durchflussgeschwindigkeit und des Druckes wird die Qualität und Quantität der Inhaltsstoffe des Filtrats beeinflusst. Die Ultrafiltration findet in einer Hohlfaser-Ultrafiltrationslage vom Typ Romicon HF (Hersteller: Romicon Inc., Woburn (MA, USA)] oder Amicon [Hersteller: Amicon Corporation, Danvers (MA, USA)] mit 4 Patronen zu je 2,5 m² Membranfläche statt. Dabei werden alle Moleküle von MG >10'000 Daltons abgetrennt. In der Stunde können 160 Liter bei 4°C ultrafiltriert werden. Durch das kontinuierliche Verdünnen wird erreicht, dass möglichst alle kleinen Moleküle filtriert werden.

Ein weiterer Vorteil des Verfahrens liegt darin, dass die drei oben besprochenen Stufen b, c und d in ein System zusammengeschlossen werden können, womit eine kontinuierliche Produktion möglich ist. Jede Anlage kann einzeln benützt oder wieder zusammen in Serie an den Wärmeaustauscher angeschlossen werden.

Das erhaltene Ultrafiltrat wird dann auf ein kleines Volumen gebracht, z.B. durch Vakuumdestillation. Vorzugsweise wird auf einen Zehntel bis einen Zwanzigstel des ursprünglichen Volumens eingeengt.

e) Elektrodialyse

In einem Elektrodialyse-Gerät (z.B. Modell BEL II von Berghof GmbH, Tübingen, BRD) werden dem eingeengten Ultrafiltrat durch Anlegen einer Potentialdifferenz in einem elektrischen Feld die gelösten Salzionen entzogen. Am schnellsten wandern die keinsten einfach geladenen Ionen, was den Vorteil einer begrenzt selektiven Arbeitsweise ermöglicht. Ebenso wandern durch die Membrane nur Ionen von MG <400. Der Entsalzungseffekt ist abhängig von der Zeit, der angelegten Potentialdifferenz, der Qualität der Membranen und der Zusammensetzung des Extraktes. Verschiedene Faktoren im Extrakt beeinflussen die theoretischen Grundlagen des Elektrodialyse-Verfahrens, so dass die optimalen Bedingungen für die Entsalzung eines Extraktes wegen den vielen Unbekannten sich nicht berechnen lassen und als Erfahrungswerte erarbeitet werden müssen. Fest steht, dass die kleinen Salzionen wie $Cl^-$ und $Na^+$ innerhalb von 2 Stunden praktisch quantitativ aus dem Extrakt entfernt werden können. Das Optimum muss aber nicht notwendigerweise bei einer 100%igen Entsalzung von $Cl^-$ und $Na^+$-Ionen liegen, denn der dabei auftretende Verlust an anderen langsam wandernden kleinen Ionen wie Aminosäuren, Peptiden usw. kann einen Verlust an biologischer Aktivität bedeuten.

Weil die Spannung nicht beliebig erhöht werden kann (Elektrolyse), müssen die Membranen nach jeder dem Gerät entsprechenden Menge dialysierten Organextraktes durch Umpolen und Waschen gereinigt werden. Es wurde auch gefunden, dass die Elektrodialyse bei einer Temperatur im Bereich von 30 bis 40°C schneller, bei etwa 37°C sogar dreimal schneller erfolgt als bei 20°C und ohne dadurch Unterschiede im Produkt zu erzeugen. Neben einer erhöhten Ionentransportaktivität wurde die Wirksamkeit der Polarisation und die Anlagerung von ungeladenen oder organischen geladenen Molekülen (englisch: das Fouling), welche die für Ionen selektive Membran verändern, herabgesetzt.

Da die für die Abtrennung der Ionen aufgewendete Energie zu einem grossen Teil in Wärme umgewandelt wird, muss zur Einhaltung einer Temperatur von 37°C nur unbedeutend Energie verwendet werden, was sich zusammen mit der Zeitverkürzung vorteilhaft für die Produktion auswirkt.

Bekanntlich ist die bisher allgemein angewendete säulenchromatographische Entsalzung sehr aufwendig und die Gefahr einer bakteriellen Kontamination, besonders bei neutralen pH-Werten, dabei sehr gross; ferner werden verschiedene biologisch aktive Substanzen bei der säulenchromatographischen Entsalzung mit der Salzfraktion abgetrennt. Daraus gehen die mit der Entsalzung durch Elektrodialyse einhergehenden Vorteile ohne weiteres hervor.

Die erfindungsgemäss erhaltenen Gesamtextrakte werden auf Sterilität, Pyrogenität und Antigenität nach den folgenden Methoden geprüft:

— Sterilität: US-Pharmacopoe XX (1980), Seiten 878—882 Europäische Pharmacopoe, 2. Auflage, 1. Nachtrag 1980, Seiten 52—55

4

— Pyrogentiät: Europäische Pharmacopoe, Band II, Schweizer Ausgabe Seiten 56—59
— Antigenität: US-Pharmacopoe XX (1980), Seite 688 (modifiziert für ein Dialysat).

Die biologischen Wirkungen der Extrakte können sowohl im Warburg-Test, durch Atmungssteigerung von Lerberhomogenisaten, als auch durch Normalisierung der Proliferation von Kulturen reversibel geschädigter Fibroblasten nachgewiesen werden. In der klinischen Prüfung zeigen der Milzgesamtextrakt und der Thymusgesamtextrakt eine regulierende bzw. normalisierende Wirkung auf das gestörte Immunsystem des Menschen.

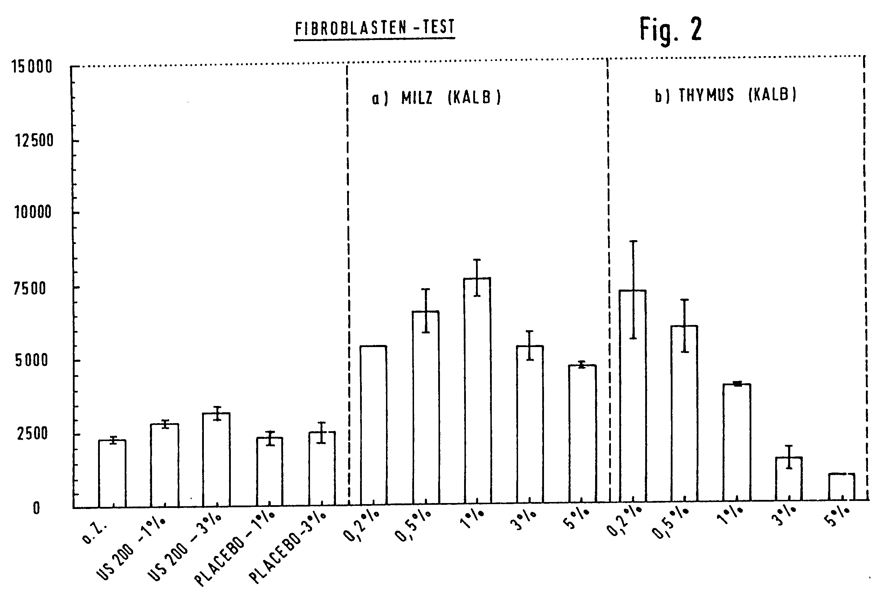

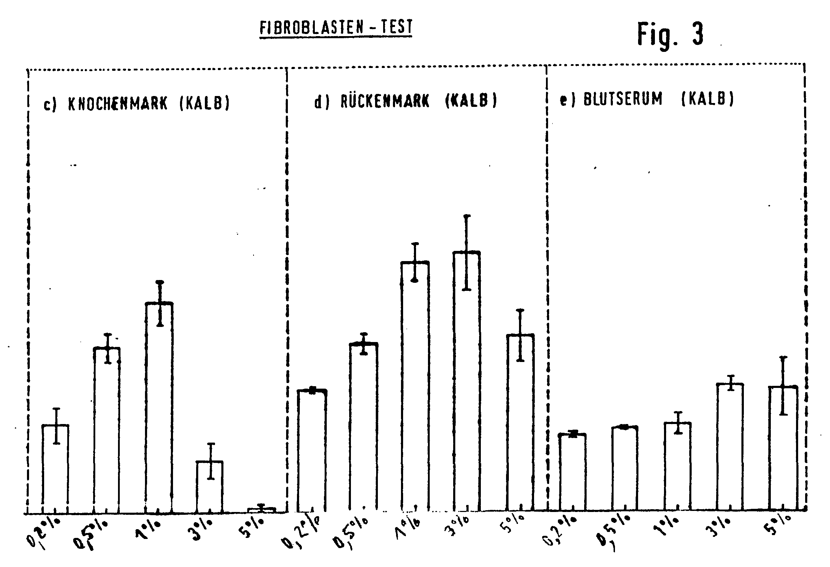

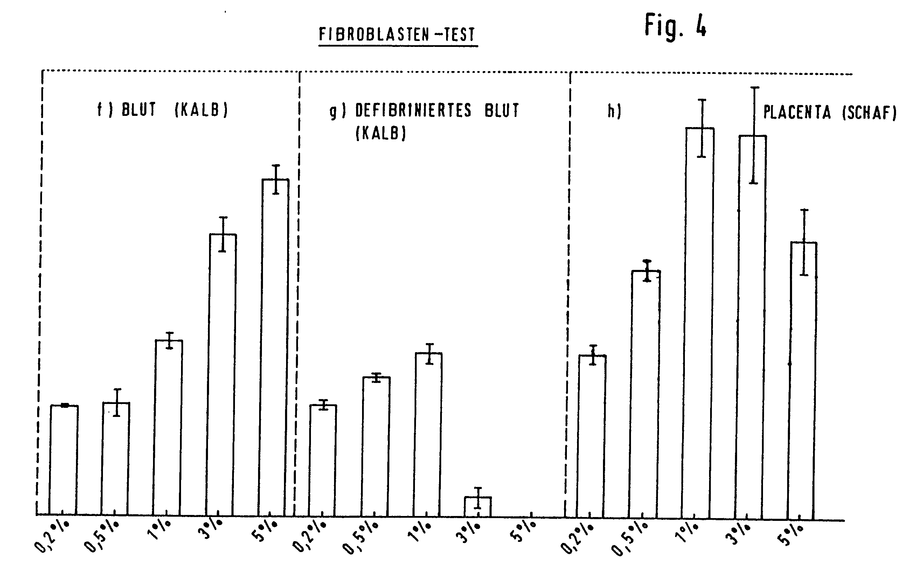

Die erwähnten biologischen Wirkungen werden für die gemäss den nachfolgenden Beispielen hergestellten Extrakte in den Tabellen 1 und 2 bzw. in den Figuren 2, 3 und 4 dargestellt:

TABELLE 1

Steigerungsfaktor des $O_2$-Verbrauchs im Warburg-Test durch Gesamtextrakt (10 mg Testsubstanz/ml)

| Messzeit | Leerwert ohne Testsubstanz | Vergleichs-präparat | Milz (Kalb) | Thymus (Kalb) | Knochenmark (Kalb) | Rückenmark (Kalb) | Blutserum (Kalb) | Blut (Kalb) | Defibriniertes Blut (Kalb) | Placenta (Schaf) |
|---|---|---|---|---|---|---|---|---|---|---|
| 30 Min. | 1 | 2,2 | 2,7 | 3,9 | 2,5 | 2,6 | 2,2 | 1,9 | 2,0 | 2,7 |
| 45 Min. | 1 | 2,3 | 2,7 | 4,0 | 2,5 | 2,7 | 2,3 | 1,9 | 2,1 | 2,8 |
| 60 Min. | 1 | 2,5 | 2,8 | 4,1 | 2,6 | 2,9 | 2,3 | 2 | 2,2 | 3,0 |

Methode: 1,1 ml Sörensenpuffer; 1,0 ml Leberhomogenisat; 0,2 ml Testlösung; 0,2 ml KOH 10%
Apparatur: Differential-Respirometer; Wasserbad: 37°C; Schüttelfrequenz 100/Min.
Leberhomogenisat: 2 g Leber per 15 ml Puffer
Test-Lösungen: 10 mg Testsubstanz per ml
Vergleichspräparat gemäss DE—PS 1.076.888

## TABELLE 2
### Steigerungsfaktor des $O_2$-Verbrauchs im Warburg-Test durch Thymus- bzw. Milzgesamtextrakt

| Messzeit | Leerwert ohne Testsubstanz | Vergleichspräparat mg/ml | | | Thymusextrakt mg/ml | | | Milzextrakt mg/ml | | |
|---|---|---|---|---|---|---|---|---|---|---|
| | | 50 | 12,5 | 6,25 | 50 | 12,5 | 6,25 | 50 | 12,5 | 6,25 |
| 30 Min. | 1 | 4,3 | 2,2 | 1,3 | 5,4 | 4,0 | 2,1 | 5,2 | 3,3 | 1,8 |
| 45 Min. | 1 | 4,6 | 2,2 | 1,2 | 5,8 | 4,0 | 1,9 | 5,6 | 3,3 | 1,6 |
| 60 Min. | 1 | 4,9 | 2,2 | 1,1 | 6,5 | 4,1 | 1,8 | 6,3 | 3,3 | 1,5 |

Methode: 1,1 ml Sörensenpuffer; 1,0 ml Leberhomogenisat; 0,2 ml Testlösung; 0,2 ml KOH 10%
Apparatur: Differential-Respirometer; Wasserbad: 37°C; Schüttelfrequenz 100/Min.
Leberhomogenisat: 2 g Leber per 15 ml Puffer
Test-Lösungen: 10 mg Testsubstanz per ml
Vergleichspräparat gemäss DE—PS 1.076.888

EP 0 140 134 B1

Die Figuren 2, 3 und 4 veanschaulichen den Einbau von $^3$H-Thymidin in die DNA von geschädigten Fibroblasten nach Zusatz von verschiedenen Testlösungen. Die Fibroblasten werden in der Wachstumsphase durch Carbonatentzug reversibel geschädigt. Durch Zusatz der Testsubstanzen wird die Proliferation der Fibroblasten, gemessen am $^3$H-Thymidin-Einbau, normalisiert.

Die Lösung des Vergleichspräparates hatte eine Konzentration von 42 mg/ml, während ale anderen Testlösungen 10 mg Trockensubstanz/ml aufwiesen. Das Vergleichspräparat wurde mit einer 1% und 3% Vol./Vol. Konzentration und alle anderen je mit 0,2; 0,5; 1; 3 und 5% Vol./Vol. getestet.

In den Figuren 2, 3 und 4 bedeutet die Abkürzung o.Z. "ohne Zusatz", US 200 stellt ein Vergleichspräparat gemäss deutscher Patentschrift 1.076.888 (K.-H. Jaeger) dar, das Placebo besteht aus einem Gemisch von Stoffen mit einem Molekulargewicht von <10'000 Daltons, welche üblicherweise im Blut von Säugetieren vorkommen.

Zur Charakterisierung der Verfahrensprodukte

Die Hochdruck- Flüssigchromatographie, auch HPLC (high performance liquid chromatography) genannt, eignet sich gut zur Charakterisierung von Organextrakten, vor allem weil die Methode eine gute Auftrennung der einzelnen Substanzen und eine gute Reproduzierbarkeit garantiert. Dadurch können Unterschiede in der Zusammensetzung verschiedener Präparate relativ leicht festgestellt werden. Die Methode erlaubt auch die Anpassung des Systems an die Aufgabenstellung. Die verwendeten Standard-Bedingungen sind wie folgt:

| | |
|---|---|
| Puffersystem: | 0,01 M $(NH_4)H_2PO_4$ |
| Mobile Phase A: | 0,01 M $(NH_4)H_2PO_4$ in 0,1%iger Propionsäure |
| Mobile Phase B: | 90% Methanol |
| Gradient: | 0% B bis 95% B |
| Zeit 30 Minuten | |
| Durchfluss: | 1,5 ml/Min. Schreiber: 10 mm/Min. |
| Wellenlänge λ: | 254 nm |
| Säule: | RP-8 (Hersteller: E. Merck AG, Darmstadt, BRD) |

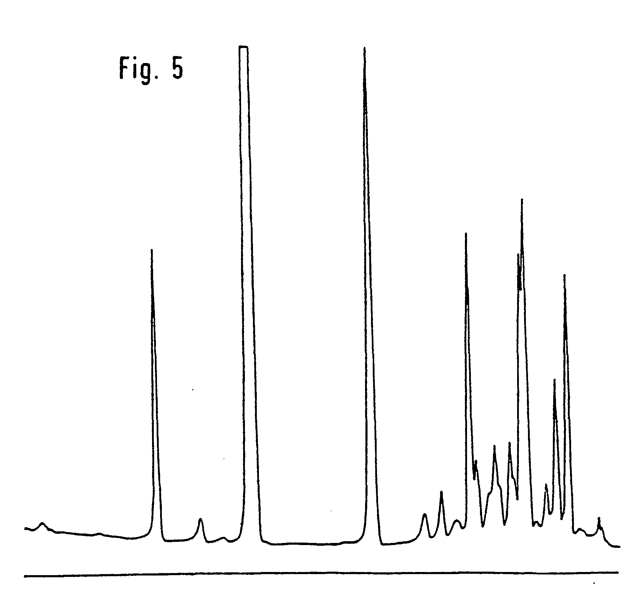

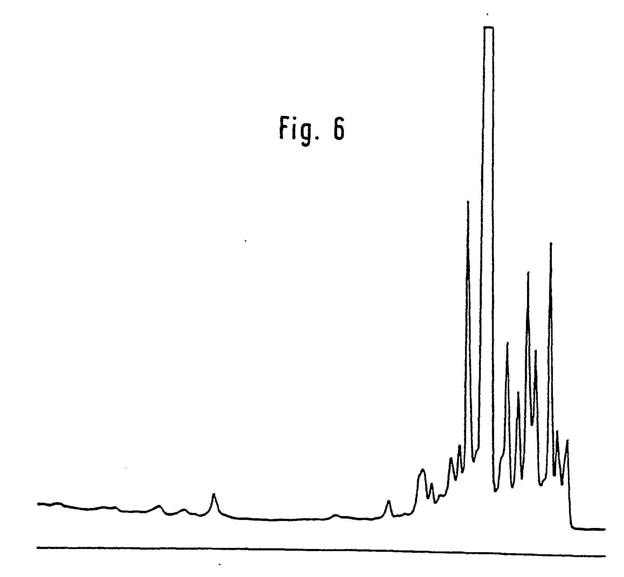

Diese Methode erlaubt, bei den erhaltenen Extrakten, beispielsweise einem Milzextrakt, zwischen dem herkömmlichen bzw. altem Dialyseverfahren gemäss deutscher Patentschrift 1.076.888 (Fig. 5) und dem erfinungsgemässen bzw. neuen Herstellungsverfahren (Fig. 6) deutliche Unterschiede nachzuweisen:

Die Diagramme unterscheiden sich besonders an zwei Stellen: Am Anfang des Diagrammes, von rechts beginnend, weist das alte Verfahren gegenüber dem neuen nur kleine Peaks auf. Die drei dominierenden Peaks in der linken Hälfte des Diagramms weisen auf die Konservierungsmittel (Nipagin/Nipasol) hin, welche beim neuen Verfahren ganz fehlen.

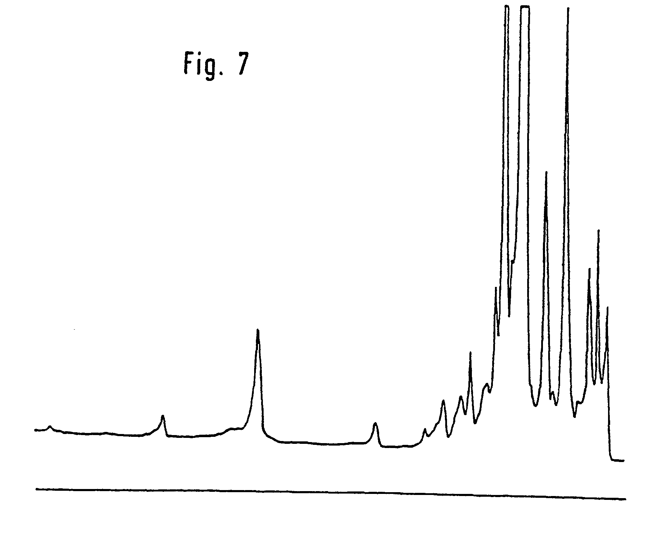

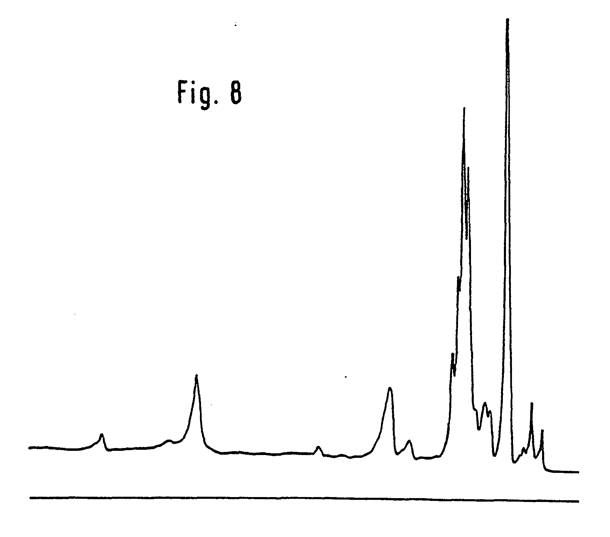

Der erfindungsgemäss hergestellte Thymusextrakt liefert ein ähnliches Elutionsprofil (Fig. 7) wie der entsprechend hergestellte Milzextrakt (Fig. 6). Nach Hydrolyse des Thymusextraktes (6 N HCL, 18 h, 110°C) verschwinden markante Peaks (Fig. 8). Durch Fraktionierung und Dünnschichtchromatograpie mit entsprechender Färbung kann nachgewiesen werden, dass die verschwundenen Peaks von nynhidrinpositiven Substanzen stammen.

Einen weiteren Beweis, dass der Peptid-Anteil in den Extrakten nach dem neuen Verfahren viel grösser ist, liefert die Isolelektrofocussierung (Auftrennung nach Massgabe des isoelektrischen Punkts). Im Extrakten, die nach dem neuen Verfahren hergestellt werden, können über 30 Comassie-Blau-positive Banden nachgewiesen werden, während beim alten nur vereinzelt schwach färbbare Banden sichtbar werden.

## Beispiel 1: Thymus

5 kg Thymus vom Kalb, sofort ohne Fettgewebe tiefgefroren und bei −25°C gelagert, werden im Fleischwolf zu einem Brei zerhackt. Dem Brei werden 5 Liter pyrogenfreies bidest. Wasser zugeführt und im Durchlauferhitzer während 10 Minuten bei 80°C grossmolekulare Anteile ausgefällt. Anschliessend wird durch Zentrifugieren (Zentrifuge, Dekanter) vom abgekühlten Brei die feste Phase abgetrennt. Dem Ueberstands werden für die kontinuierliche Ultrafiltration nochmals 10 Liter pyrogenfreies Wasser zugeführt (Membranfläche 2,5 m², PM-10 System Romicon HF 1/3 S). Dem eingeengten Ultrafiltrat werden durch Elektrodialyse Salzionen entzogen. Es wird ein salzarmer, pyrogen- und antigenfreier Thymusextrakt erhalten.

## Beispiel 2: Milz

a) 5 kg frische Milzorgane von gesunden Kälbern werden ohne Fettgewebe sofort tiefgefroren. Die tiefgefrorenen Blöcke werden im Fleischwolf zu einem homogenen Brei verarbeitet, der zusammen mit 6 Liter pyrogenfreiem Wasser im Durchlauferhitzer (Rohrlänge 5 m) innerhalb von 3 Minuten auf 80°C erhitzt wird und im Kreislauf während 8 Minuten auf gleichbleibender Temperatur von 80°C gehalten wird. Nach Abkühlung auf 4°C innerhalb von 6 Minuten mit dem Durchlaufkühler werden die ausgefällten Stoffe durch Zentrifugation abgetrennt. Der Ueberstand wird mit 18 Liter pyrogenfreiem Wasser kontinuierlich ultrafiltriert (MG < 10'000 Daltons). Die Salzionen werden dem Konzentrat durch Elektrodialyse weitgehend entzogen. Die nach allen Stufen des Verfahrens durchgeführten Teste zur Bestimmung des Wachstums von Mikroorganismen waren negativ.

b) 250 kg frische Milzorgane von gesunden Kälbern werden (ohne Fettgewebe) sofort tiefgefroren. Die tiefgefrorenen Blöcke werden im Fleischwolf zu einem homogenen Brei verarbeitet, der anschliessend zusammen mit 250 Liter pyrogenfreiem Wasser in einem Durchlauferhitzer (Rohrlänge 20 m) während 5 Minuten (5 Min. = Verweildauer eines Teilchens im Rohr) auf 80°C erhitzt wird, und dann im Durchlaufkühler innerhalb von 6 Minuten auf 4°C abgekühlt. Im angeschlossenen Dekanter werden die festen ausgefällten. Substanzen abgetrennt. Der wässrige Ueberstand wird kontinuierlich durch Zugabe von 900 Liter pyrogenfreiem Wasser ultrafiltriert (MG < 10'000 Daltons). Das Ultrafiltrat wird in einem Umlaufverdampfer eingeengt und anschliessend sterilfiltriert. Dem Konzentrat werden portionenweise in einer Elektrodialyse Salzionen entzogen, so dass eine salzarme wässrige Lösung von kleinmolekularen Substanzen aus der Milz entsteht. Das Verfahren erfolgt in einem geschlossenen System.

## Beispiel 3: Kalbsblutserum

Zu 6 Liter Serum aus frischem Kälberblut werden 3 Liter pyrogenfreies Wasser zugegeben. Diese Suspension wird im Durchlauferhitzer auf 75°C erhitzt, während 5 Minuten bei gleichbleibender Temperatur gehalten und anschliessend durch das gleiche Rohrsystem auf 4°C abgekühlt. Die ausgefällten Stoffe werden durch Zentrifugation abgetrennt. Der Ueberstand von 5,5 Liter wird mit 15 Liter pyrogenfreiem Wasser kontinuierlich ultrafiltriert (MG < 10'000 Daltons). Nach weitgehender Entfernung der Salze durch Elektrodialyse wird eine sterile und pyrogenfreie Lösung erhalten. Dieser kleinmolekulare Serum-Extrakt bewirkt bei in Kultur reversibel geschädigten Fibroblasten eine Normalisierung der Proliferation.

## Beispiel 4: Frisches Kälberblut

5 Liter frisches Kälberblut werden mit 5 Liter pyrogenfreiem Wasser vermischt und im Durchlauferhitzer auf 80°C erhitzt. Nach 3 Minuten gleichbleibender Temperatur wird die Suspension im Durchlaufkühler auf 4°C abgekühlt und die ausgefällten Produkte durch Zentrifugierung entfernt. Der Ueberstand von 6 Litern wird mit 18 Liter pyrogenfreiem Wasser kontinuierlich ultrafiltriert (MG < 10'000 Daltons). Nach weitgehender Eliminierung der Salze durch Elektrodialyse wird eine sterile und pyrogenfreie Lösung erhalten. Der auf diese Weise gewonnene kleinmolekulare Kälberblut-Extrakt bewirkt bei in Kultur geschädigten Fibroblasten eine Normalisierung der Proliferation.

## Beispiel 5: Defibriniertes Kalbsblut

5,1 kg defibriniertes Kälberblut wird mit 5 Liter pyrogenfreiem Wasser im Durchlauferhitzer auf 80°C erhitzt und während 5 Minuten bei gleich hoher Temperatur gehalten. Anschliessend wird der Brei im Durchlaufkühler auf 4°C abgekühlt und die Ausfällungen durch Zentrifugation abgetrennt. Der Ueberstand von 7 Litern wird kontinuierlich mit 18 Litern pyrogenfreiem Wasser ultrafiltriert (MG < 10'000 Daltons). Nach erfolgter Elektrodialyse erhält man eine salzarme, sterile und pyrogenfreie, aufkonzentrierte Lösung. Die Lösung bewirkt bei in Kultur reversibel geschädigten Fibroblasten eine Normlisierung der Proliferation.

## Beispiel 6: Rückenmark vom Kalb

Aus Wirbelknochen von frisch geschlachteten gesunden Kälbern werden 5 kg Mark isoliert und mit 5 Liter pyrogenfreiem Wasser im Durchlauferhitzer auf 80°C erhitzt und während 5 Minuten auf gleichbleibender Temperatur gehalten. Anschliessend wird der Brei im Durchlaufkühlter auf 4°C abgekühlt. Nach der Zentrifugation kann 1/3 des Volumens als Ueberstand direkt für die nächste Stufe verwendet werden. Die 3 Liter werden mit 9 Liter pyrogenfreiem Wasser ultrafiltriert (MG < 10'000 Daltons). Dem eingeengten Konzentrat werden durch Elektrodialyse Salzionen entzogen. Die salzarme Lösung war steril und pyrogenfrei. Sie bewirkte bei in Kultur reversibel geschädigten Fibroblasten eine Normalisierung der Proliferation und in Knochenmarkzellen eine Erhöhung der Aktivität der terminalen Desoxytransferase.

## Beispiel 7: Mark aus Femur-Knochen

Brei von 3,5 kg Mark aus 30 kg Femur-Knochen von frisch geschlachteten Kälbern werden mit 5 Liter pyrogenfreiem Wasser im Durchlauferhitzer auf 75°C erhitzt. Die Suspension wird während 5 Minuten auf 75°C gehalten. Nach der Abkühlung im Durchlaufkühler auf 4°C wird das erstarrte Fett von der übrigen Lösung abgetrennt. Nach der Zentrifugation der entfetteten Lösung wird der Ueberstand von 5 Litern mit 15 Litern pyrogenfreiem Wasser kontinuierlich ultrafiltriert (MG < 10'000 Daltons). Dem eingeengten Präparat werden durch Elektrodialyse Salzionen entzogen. Die salzarme Lösung ist steril und pyrogenfrei. Sie bewirkt bei in Kultur reversibel geschädigten Fibroblasten eine Normalisierung der Proliferation und eine Erhöhung der Aktivität der terminalen Desoxytransferase.

## Beispiel 8: Schafsplacenta

4,700 kg Brei aus tiefgefrorenen Rosetten von frischer Schafsplacenta wird mit 5 Liter pyrogenfreiem Wasser im Durchlauferhitzer auf 80°C erhitzt und während 5 Minuten im Kreislauf auf 80°C gehalten. Nach der Abkühlung auf 4°C wird die Ausfällung durch Zentrifugation abgetrennt und der Ueberstand von 7 Litern mit 10 Liter pyrogenfreiem Wasser kontinuierlich ultrafiltriert (MG < 10'000 Daltons). Die durch Elektrodialyse erhaltene salzarme Lösung war steril und pyrogenfrei. Sie bewirkt bei in Kultur reversibel geschädigten Fibroblasten eine Normalisierung der Proliferation.

### Beispiel 9: Thymozyten

10 kg frische Thymusorgane vom Kalb werden mit 10 Liter Phosphat-gepufferter Kochsalzlösung (pH 7,2) in einem feinmaschigen Sieb mit einem Gummistopfen zerrieben. Dadurch werden die Thymozyten aus dem Organgewebe gelöst; die zurückbleibenden Gewebeteile werden verworfen. Die Thymozyten werden anschliessend 2mal mit Phosphat-gepufferter Kochsalzlösung gewaschen und anschliessend in 10 Liter bidestilliertem, pyrogenfreiem Wasser ausgenommen ($18 \times 10^8$ Zellen/ml). Die Thymozyten werden in 100 ml Portionen mit Ultraschall (Soniprep 150: Amplitude 4, mittlere Frequenz) während 1 Minute aufgeschlossen und zerkleinert. Der Thymocytenbrei wird mit weiteren 10 Litern bidestilliertem, pyrogenfreiem Wasser im Durchlauferhitzer auf 80°C erwärmt und nach 10 Minuten wieder auf 10°C abgekühlt. Die denaturierten Proteine und andere unlösliche Zellfragmente werden durch Zentrifugation in einer Sorvall-Zentrifuge mit Durchlaufrotor TZ-28 (20'000 RPM, Verweilzeit 10 Min.) abgetrennt. Anschliessend wird der Ueberstand mit weiteren 20 Litern bidestilliertem, pyrogenfreiem Wasser kontinuierlich ultrafiltriert (Ausschlussgrenze MG > 10'000). Das Ultrafiltrat wird auf 2'000 ml eingeengt und elektrodialysiert. Das peptidhaltige Produkt zeigt bei $CO_2$-geschädigten Fibroblasten eine wachstumsfördernde Aktivität.

### Beispiel 10: Fetale Kälbernierenzellen

Epithelzellen von fetalen Kälbernieren (FKNE) werden in Rollflaschen zu 850 ml mit dem "Minimal Essential Medium" [Science *130*, 432 (1959)] ind 7% fetalem Kalbsserum kultiviert. Kurz bevor ein geschlossener Zellrasen gebildet wird, werden die Zellrasen mit Phosphatgepufferter Kochsalzlösung (pH 7,2) gewaschen und mit Ethylendiamintetraacetat und Wasser sowie einem Gummischaber aus den Rollflaschen gelöst und in insgesamt 4 Liter bidestilliertem, pyrogenfreiem Wasser aufgenommen. Die FKNE-Zellen ($4 \times 10^8$/ml) werden in 100 ml Portionen mit Ultraschall (Soniprep 150: Amplitude 4, mittlere Frequenz) während 1 Minute aufgeschlossen und zerkleinert. Der FKNE-Zellbrei wird mit weiteren 4 Litern Wasser im Durchlauferhitzer auf 80°C erwärmt und nach 10 Minuten wieder auf 10°C abgekühlt. Die denaturierten Proteine und andere unlösliche Zellfragmente werden durch Zentrifugation in einer Sorvall-Zentrifuge mit Durchlaufrotor TZ-28 (20'000 RPM, Verweilzeit 10 Min.) abgetrennt. Anschliessend wird der Ueberstand mit weiteren 8 Litern bidestilliertem, pyrogenfreiem Wasser kontinuierlich ultrafiltriert (Ausschlussgrenze MG > 10'000). Das Ultrafiltrat wird auf 500 ml eingeengt und elektrodialysiert. Das peptidhaltige Produkt zeigt bei $CO_2$-geschädigten Fibroblasten eine wachstumsfördernde Aktivität.

## Patentansprüche

1. Verfahren zur Herstellung eines biologisch aktiven, salzarmen, pyrogenfreien, sterilen und antigenfreien Gesamtextraktes aus Organen von Säugetieren und aus Zellkulturen, bestehend aus einem Gemisch biologisch wirksamer Substanzen mit einem Molekulargewicht unter 10'000 Daltons, dadurch gekennzeichnet, dass man unter Ausschluss jeglichen fremden Stoffes ausser Wasser und ohne Verwendung jeglichen Trägermaterials für Trennmethoden das unter keimarmen bzw. sterilen Bedingungen beschaffene und allenfalls gelagerte Ausgangsmaterial unter Aufschluss der Zellen zerkleinert, das zerkleinerte und aufgeschlossene Material in einem als Wärmeaustauscher fungierenden Durchlaufsystem mit hintereinander arbeitendem Wärmeträger und Kühlmedium rasch auf eine Temperatur von 70 bis 90°C erhitzt und rasch wieder auf niedrige Temperatur abkühlt, die dadurch ausgefällten Produkte von der Lösung durch Zentrifugation abtrennt, aus der Lösung durch Ultrafiltration die Substanzen mit einem Molekulargewicht über 10'000 Daltons entfernt und der verbleibenden Lösung durch Elektrodialyse die Salzionen weitgehend entzieht.

2. Verfahren nach Anspruch 1, dadurch gekennzeichnet, dass als Ausgangsmaterial der Thymus, die Milz, die Leber, das Herz, die Plazenta, das Knochenmark oder das Blut von Säugetieren verwendet wird.

3. Verfahren nach Anspruch 2, dadurch gekennzeichnet, dass als Ausgangsmaterial der Thymus verwendet wird.

4. Verfahren nach Anspruch 1, dadurch gekennzeichnet, dass als Ausgangsmaterial Kulturen von Thymozyten, von fetalen Kälbernierenzellen, von Leukozyten, von Hybridzellen, von Fibroblasten oder aus den entsprechenden Kulturen gewonnene Zellmassen verwendet werden.

5. Verfahren nach Anspruch 1, dadurch gekennzeichnet, dass die Zerkleinerung und der Aufschluss der Zellkulturen durch Einwirkung von Ultraschall ausgeführt wird.

6. Verfahren nach Anspruch 1, dadurch gekennzeichnet, dass die Elektrodialyse bei einer Temperatur im Bereich von 30 bis 40°C ausgeführt wird.

7. Verfahren nach Anspruch 1, dadurch gekennzeichnet, dass die Stufen der Hitzeeinwirkung, der Abtrennung der ausgefällten Produkte und der Ultrafiltration in kontinuierlichem Verfahren ausgeführt werden.

8. Verfahren nach Anspruch 1, dadurch gekennzeichnet, dass der erhaltene Gesamtextrakt zusätzlich einer Sterilisation unterworfen wird.

# EP 0 140 134 B1

**Revendications**

1. Procédé de préparation d'un extrait total, biologiquement actif, pauvre en sels, exempt de substances pyrogènes, stérile et exempt d'antigènes, à partir d'organes de mammifères et de cultures cellulaires, consistant en un mélange de substances biologiquement actives ayant un poids moléculaire inférieur à 10000 Daltons, caractérisé en ce qu'on broie avec désagrégation des cellules, en excluant tout corps étranger sauf l'eau et sans utiliser aucun matériel de support pour des méthodes de séparation, le produit de départ qui a été procuré et éventuellement entreposé en des conditions pauvres en germes ou stériles, on chauffe rapidement à une température de 70 à 90°C le produit broyé et désagrégé dans un dispositif de coulage fonctionnant comme un échangeur de chaleur, comprenant une source de chaleur et un milieu réfrigérant opérant l'un derrière l'autre, et on refroidit rapidement à basse température, on sépare de la solution, par centrifugation, les produits ainsi précipités, on élimine de la solution, par ultrafiltration, les substances ayant un poids moléculaire supérieur à 10000 Daltons et on enlève de la solution restante, par électrolyse, une grande partie des ions de sel.

2. Procédé selon la revendication 1, caractérisé en ce qu'on utilise comme produit de départ le thymus, le foie, le coeur, le placenta, la moelle osseuse ou le sang de mammifères.

3. Procédé selon la revendication 2, caractérisé en ce qu'on utilise comme product de départ le thymus.

4. Procédé selon la revendication 1, caractérisé en ce qu'on utilise comme produit de départ des cultures de thymocytes, de cellules fétales de reins de veau, de leucocytes, de cellules hybrides, de fibroblastes ou des masses cellulaires obtenues à partir de cultures correspondantes.

5. Procédé selon la revendication 1, caractérisé en ce que l'on effectue le broyage et la désagrégation des cultures cellulaires par action d'ultrasons.

6. Procédé selon la revendication 1, caractérisé en ce que l'on effectue l'électrodialyse à une température dans le domaine de 30 à 40°C.

7. Procédé selon la revendication 1, caractérisé en ce que l'on effectue les étapes de chauffage, séparation des produits précipités et ultrafiltration en procédé continu.

8. Procédé selon la revendication 1, caractérisé en ce qu'on soumet l'extrait total obtenu, en outre, à une stérilisation.

**Claims**

1. A process for the manufacture of a biologically active, low-salt, pyrogen-free, sterile and antigen-free total-extract made from mammal organs and from cell cultures and composed of a mixture of biologically active substances with a molecular weight below 10,000 daltons, characterized in that the starting material, which is supplied and, if necessary, stored under germ-free or sterile conditions, is, with the exclusion of any foreign substances, except water, and without the use of any carrier material for separating methods, comminuted with the breaking up of the cells, the comminuted and broken-up material is then quickly heated to a temperature of 70 to 90°C in a flow-through system acting as a heat exchanger and having heat carriers and cooling media working subsequent to one another, and is then quickly cooled to a low temperature, the products thereby precipitated are separated from the solution by centrifugation, the substances with a molecular weight above 10,000 daltons are removed from the solution by ultrafiltration and the salt ions are substantially removed from the remaining solution by means of electrodialysis.

2. A process in accordance with Claim 1, characterized in that thymus, spleen, liver, heart, placenta, bone marrow or mammalian blood are used as a starting material.

3. A process in accordance with Claim 2, characterized in that thymus is used as a starting material.

4. A process in accordance with Claim 1, characterized in that cultures of thymocytes, of foetal calf-kidney cells, of leucocytes, of hybrid cells, of fibroblasts or cell masses obtained from the corresponding cultures are used.

5. A process in accordance with Claim 1, characterized in that the comminution is carried out by the action of ultrasonics.

6. A process in accordance with Claim 1, characterized in that the electrodialysis takes place at a temperature in the range of 30 to 40°C.

7. A process in accordance with Claim 1, characterized in that the stages of thermal action, separation of the precipitated products and ultrafiltration are carried out in a continuous process.

8. A process in accordance with Claim 1, characterized in that in addition the total-extract obtained is subjected to sterilization.

11

# Fig. 1

HYDROPHILE SUBSTANZEN    HYDROPHOBE SUBSTANZEN

SALZIONEN

TEIL A    TEIL B

Fig. 2

FIBROBLASTEN-TEST

a) MILZ (KALB)   b) THYMUS (KALB)

FIBROBLASTEN - TEST

Fig. 3

c) KNOCHENMARK (KALB)

d) RÜCKENMARK (KALB)

e) BLUTSERUM (KALB)

EP 0 140 134 B1

FIBROBLASTEN-TEST

Fig. 4

f) BLUT (KALB)

g) DEFIBRINIERTES BLUT (KALB)

h) PLACENTA (SCHAF)

0,2% 0,5% 1% 3% 5% 0,2% 0,5% 1% 3% 5% 0,2% 0,5% 1% 3% 5%

EP 0 140 134 B1

Fig. 5

Fig. 6

Fig. 7

Fig. 8